# EUROPEAN PATENT SPECIFICATION

(11) **EP 2 329 782 B1**
(45) Date of publication and mention of the grant of the patent: **21.05.2014**
(21) Application number: 09817859.3
(22) Date of filing: 01.10.2009
(51) Int. Cl.: A61B 18/12, A61B 18/14, A61B 18/00

(54) **ELECTROSURGICAL DEVICE**
ELEKTROCHIRURGISCHE VORRICHTUNG
DISPOSITIF ÉLECTROCHIRURGICAL

(30) Priority: 03.10.2008 US 244920; 15.10.2008 US 252054
(43) Date of publication of application: 08.06.2011
(73) Proprietor: Olympus Medical Systems Corp., Tokyo 151-0072 (JP)
(72) Inventor: MIHORI Takashi, Tokyo 151-0072 (JP); KABAYA, Akinori, Tokyo 151-0072 (JP)
(74) Representative: Gunzelmann, Rainer
(86) International application number: PCT/JP2009/067155
(87) International publication number: WO 2010/038827

(56) References cited:
- WO-A1-2008/142404
- JP-A- H08 512 229
- JP-A- 2001 029 356
- JP-A- 2002 537 938
- JP-A- 2004 512 135
- JP-A- 2008 510 507
- JP-A- 2009 045 456
- US-A1- 2007 129 726

## Description

### Technical Field

The present invention relates to an electrosurgical apparatus.

### Background Art

Conventionally, an electrosurgical apparatus such as a cautery knife is used to perform a treatment such as dissection, coagulation or hemostasis on a living tissue in a surgical operation or the like. Such an electrosurgical apparatus is configured by being provided with a high frequency power supply which outputs high frequency electric power, and a treatment instrument connected to the high frequency power supply.

It is known that in a case where coagulation or hemostasis on a living tissue for example is performed by using the electrosurgical apparatus configured as described above, the state of dehydration of an objective portion and the state of joining in the objective portion relate closely to each other. That is, if the state of dehydration of the objective portion is closer to the completely dehydrated state, the reliability of joining state in the objective portion is higher.

In addition, for example, an apparatus proposed in Japanese Patent Application Laid-Open Publication No. 2008-36439 is widely known as an apparatus having a configuration similar to that of the above-described electrosurgical apparatus.

Further, in a high-frequency treatment apparatus, a living tissue is held by a pair of electrodes and the held living tissue is treated by applying a high frequency wave thereto. In such a high-frequency treatment apparatus, an impedance of the held living tissue is measured and a current value, a voltage value, a frequency, etc. of the high-frequency wave supplied to the living tissue are controlled based on the measured impedance to thereby perform an appropriate treatment. For example, various control methods for performing appropriate coagulation and cutting treatment are disclosed in Japanese Patent Application Laid-Open Publication Nos. 2001-269353 , 2001-29355 , 2000-107197 , 2001-252284 .

Incidentally, taking account of a fact that a vapor layer is formed inside the living tissue as the dehydration advances so that the impedance of the living tissue rises, there may occur a circumstance where the dehydration does not advance since an electric power applied to the living tissue is lowered as time elapses.

Further, taking account of the fact that a vapor layer is formed inside the living tissue as the dehydration advances so that the impedance of the living tissue raises, it is considered that a dehydration state of the living tissue can not be detected precisely by merely detecting the rise of the impedance of the living tissue.

On the other hand, according to a technique as disclosed in Japanese Patent Application Laid-Open Publication No. 2008-36439 , because of mere determination of an output power etc. based on a tissue impedance response at a very initial stage, there causes a problem that it results in that the living tissue at the objective portion is not sufficiently dehydrated and that a coapting force of the objective portion is weakened.

Also, in the technique as disclosed in Japanese Patent Application Laid-Open Publication Nos. 2001-269353 , 2001-29355 , 2000-107197 , 2001-252284 , there is caused a problem that the living tissue of the objective portion is not dehydrated sufficiently and the coapting force of the objective portion is weakened.

Document US 2007/0129726 A1 discloses an electrode structure and a mechanism for automated or user-selected operation or compensation of the electrodes to determine tissue coverage and/or prevent arcing between bottom electrodes during electrocautery.

The present invention has been made in view of the above-mentioned circumstances and an object of the invention is to provide an electrosurgical apparatus which is capable of securely dehydrating the living tissue of the objective portion.

### Disclosure of Invention

### Means for Solving the Problem

An electrosurgical apparatus in the present invention includes the features of claim 1.

A high-frequency treatment apparatus not forming part of the present invention comprises: a treatment high-frequency wave output unit capable of outputting a treatment high-frequency wave to be applied to a treatment target living tissue; a detection unit for detecting an impedance of the treatment target living tissue; and a control unit capable of controlling the treatment high-frequency wave output unit based on a detection result of the detection unit, the control unit setting a treatment frequency of the treatment high-frequency wave to a first treatment frequency for treating the living tissue mainly by Joule heat when the impedance of the treatment target living tissue is smaller than a predetermined threshold value, and to a second treatment frequency higher than the first treatment frequency for treating the living tissue mainly by high-frequency induction heating when the impedance of the treatment target living tissue is larger than the predetermined threshold value.

A high-frequency treatment method not forming part of the present invention, comprising: performing detection of an impedance of a treatment target living tissue; performing a first treatment for treating the living tissue mainly by Joule heat by applying a treatment high-frequency wave having a first treatment frequency to the living tissue when the impedance of the treatment target living tissue is smaller than a predetermined threshold; and performing a second treatment for treating the living tissue mainly by high-frequency induction heating by applying a treatment high-frequency wave having a second treatment frequency which is higher than the first treatment frequency to the lining tissue when the impedance of the treatment target living tissue is larger than the predetermined threshold.

### Brief Description of the Drawings

Fig. 1 is a diagram showing the configuration of an essential portion of an electrosurgical apparatus according to a first embodiment of the present invention;
Fig. 2 is a diagram showing changes with respect to time in the value of impedance of a living tissue;
Fig. 3 is a diagram showing changes with respect to time in the value of electric power applied to the living tissue;
Fig. 4 is a diagram showing a state where the frequency of high frequency power applied to the living tissue changes stepwise;
Fig. 5 is a diagram showing an example of processing performed in the electrosurgical apparatus shown in Fig. 1; and
Fig. 6 is a diagram showing the correlation between the frequency and the electric conductivity of the living tissue.
Fig. 7 is a block diagram showing a high-frequency treatment apparatus according to a second embodiment not forming part of the present invention;
Fig. 8 is a schematic diagram showing a circuit model of a cable and the living tissue according to the second embodiment;
Fig. 9 is a flowchart showing a high-frequency wave control method according to the second embodiment;
Fig. 10 is a graph showing variation of an output power with respect to impedance in the high-frequency wave control method according to the second embodiment;
Fig. 11 is a graph showing variation of the impedance with respect to time in the high-frequency wave control method according to the second embodiment;
Fig. 12 is a graph showing variation of the output power with respect to time in the high-frequency wave control method according to the second embodiment;
Fig. 13 is a graph showing variation of the output power with respect to time in the high-frequency wave control method according to the second embodiment;
Fig. 14 is a graph showing variation of a frequency with respect to time in the high-frequency wave control method according to the second embodiment;
Fig. 15 is a graph showing variation of a calorific value by Joule heat and by a high-frequency induction heating with respect to time in the high-frequency wave control method according to the second embodiment;
Fig. 16 is a block diagram showing a high-frequency treatment apparatus according to a third embodiment not forming part of the present invention;
Fig. 17 is a flowchart showing a high-frequency wave control method according to the third embodiment;
Fig. 18 is a timing chart showing a treatment high-frequency wave and a detection high-frequency wave in the high-frequency wave control method according to the third embodiment;
Fig. 19 is a timing chart showing a treatment high-frequency wave and a detection high-frequency wave in the high-frequency wave control method according to a fourth embodiment not forming part of the present invention;
Fig. 20 is a timing chart showing a treatment high-frequency wave and a detection high-frequency wave in the high-frequency wave control method according to a fifth embodiment not forming part of the present invention;
Fig. 21A is a front view showing electrodes according to a first reference embodiment of the present invention;
Fig. 21B is a cross-sectional view showing the electrodes according to the first reference embodiment of the present invention;
Fig. 22 is a front view showing electrodes according to a second reference embodiment of the present invention; and
Fig. 23 is a front view showing electrodes according to a third reference embodiment of the present invention.

### Best Mode for Carrying Out the Invention

Embodiments of the present invention will be described with reference to the accompanying drawings.

### (First embodiment)

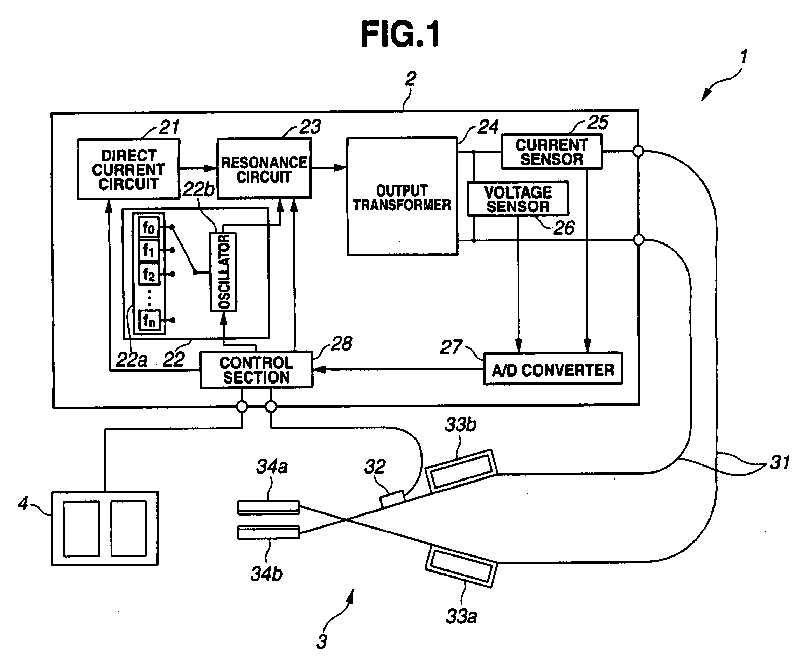

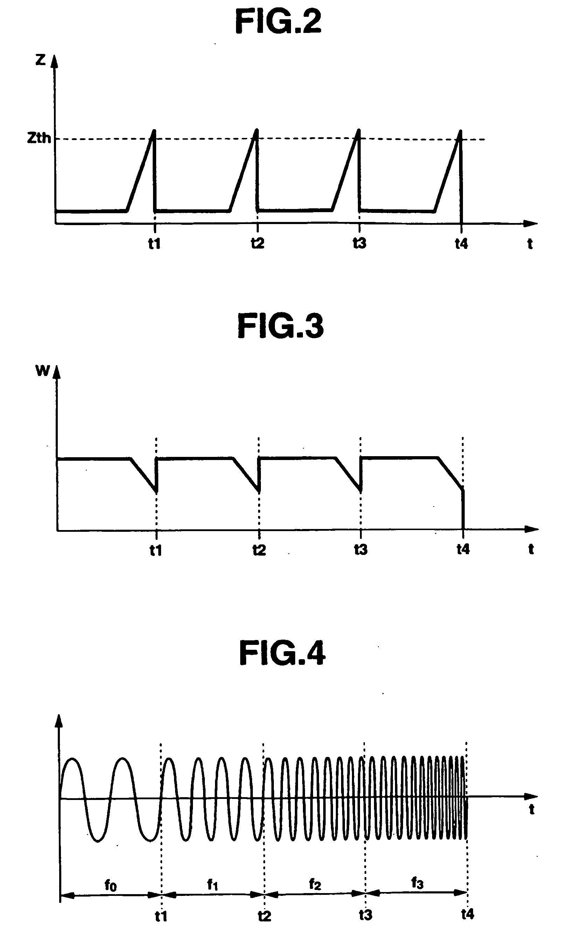

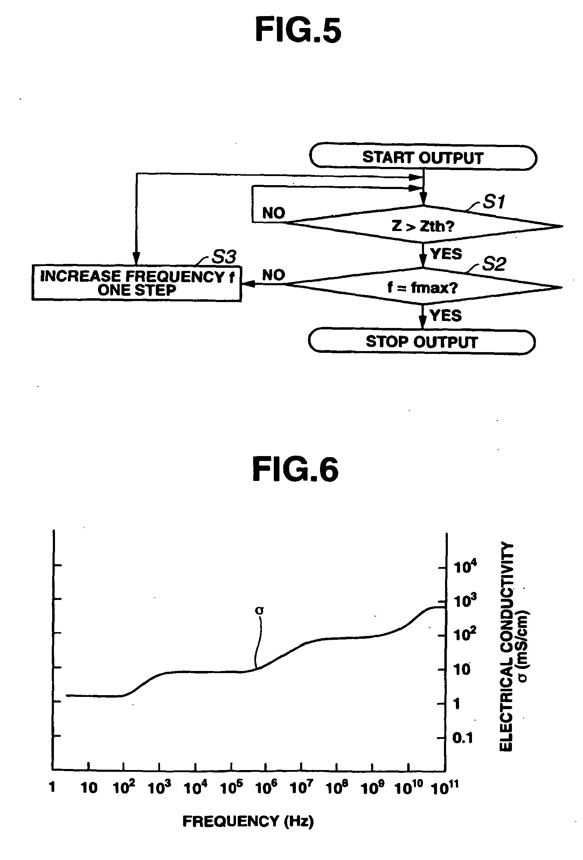

Figs. 1 to 6 relate to a first embodiment according to the present invention. Fig. 1 is a diagram showing the configuration of an essential portion of an electrosurgical apparatus according to the first embodiment of the present invention. Fig. 2 is a diagram showing changes with respect to time in the value of impedance of a living tissue. Fig. 3 is a diagram showing changes with respect to time in the value of electric power applied to the living tissue. Fig. 4 is a diagram showing a state where the frequency of high frequency power applied to the living tissue changes stepwise. Fig. 5 is a diagram showing an example of processing performed in the electrosurgical apparatus shown in Fig. 1. Fig. 6 is a diagram showing the correlation between the frequency and the electric conductivity of the living tissue.

As shown in Fig. 1, an electrosurgical apparatus 1 is configured to have a high frequency power supply 2 which outputs high frequency power, and a treatment instrument 3 which can be connected to the high frequency power supply 2 via a cable 31, and with which a treatment on a living tissue 101 is performed by supplying to the living tissue 101 high frequency power outputted from the high frequency power supply 2. A foot switch 4 provided as an operation command section for supplying a command to the high frequency power supply 2 is also connected to the high frequency power supply 2.

The treatment instrument 3 is provided with a hand switch 32 for outputting a command signal for outputting high frequency power from the high frequency power supply 2. For example, the hand switch 32 outputs the command signal only during a time period during which the switch is pressed by the action of an operator's hand or the like.

The foot switch 4 has such a configuration as to output a command signal for outputting high frequency power from the high frequency power supply 2. For example, the foot switch 4 outputs the command signal only during a time period during which the switch is pressed by the action of an operator's foot or the like.

The high frequency power supply 2 is configured to have a direct current power supply circuit 21, a waveform generation circuit 22, a resonance circuit 23, an output transformer 24, a current sensor 25, a voltage sensor 26, an A/D converter 27, and a control section 28.

The direct current power supply circuit 21 converts power supplied from a commercial power supply or the like not shown in the figure into direct current power having a signal level according to control performed by the control section 28, and outputs the direct current power to the resonance circuit 23.

The waveform generation circuit 22 detects a switching frequency set by the control section 28 and outputs a timing signal according to the switching frequency to the resonance circuit 23.

More specifically, the waveform generation circuit 22 is configured to have an oscillation source 22a and an oscillator 22b.

In the oscillation source 22a, (n + 1) types of frequencies consisting of frequencies f₀, f₁, f₂, ..., fₙ are stored.

The oscillator 22b selects one frequency coinciding with the switching frequency set by the control section 28 from the (n + 1) types of frequencies stored in the oscillation source 22a. The oscillator 22b then generates a timing signal according to the one frequency and outputs the timing signal to the resonance circuit 23.

The resonance circuit 23 has such a configuration as to be able to freely change its inductance value and capacitance value according to control performed by the control section 28. After the resonance circuit 23 has changed its inductance value and capacitance value according to control performed by the control section 28, it generates and outputs high frequency power on the basis of the direct current power outputted from the direct current power supply circuit 21 and the timing signal outputted from the waveform generation circuit 22. That is, high frequency power generated by the resonance circuit 23 has a signal level according to the direct current power outputted from the direct current power supply circuit 21 and having a frequency according to the timing signal outputted from the waveform generation circuit 22.

When a high frequency voltage having a predetermined frequency and a peak value V1 is applied to the primary-side circuit of the transformer 24, the transformer 24 generates in its secondary-side circuit a high frequency voltage having the predetermined frequency and a peak value V2 by electromagnetic induction. When the high frequency voltage having the predetermined frequency and a peak value V2 is generated in the secondary-side circuit of the transformer 24, a high frequency current based on this high frequency voltage and having the predetermined frequency and a peak value I2 is outputted to the treatment instrument 3 connected to the high frequency power supply 2 via the current sensor 25 and the cable 31. Also, when the high frequency voltage having the predetermined frequency and a peak value 12 is generated in the secondary-side circuit of the transformer 24, a high frequency voltage based on the high frequency current and having the predetermined frequency and a peak value V2 is outputted to the treatment instrument 3 connected to the high frequency power supply 2 via the voltage sensor 26 and the cable 31.

The voltage sensor 26 detects a value according to the peak value V2 of the high frequency voltage generated in the secondary-side circuit of the transformer 24 and outputs a value according to the detected peak value V2 as output voltage value information to the A/D converter 27.

The current sensor 25 detects a value according to the peak value I2 of the high frequency current generated based on the high frequency voltage in the secondary-side circuit of the transformer 24 and outputs a value according to the detected peak value I2 as output current value information to the A/D converter 27.

The A/D converter 27 converts each of the output voltage value information outputted from the voltage sensor 26 and the output current value information outputted from the current sensor 25 from analog form into digital form and outputs the converted information to the control section 28.

The control section 28 constituted by a CPU and other components continuously performs control for supplying high frequency power to the treatment instrument 3 while the command signal is being outputted from at least one of the hand switch 32 and the foot switch 4.

The control section 28 computes the impedance value Z of a living tissue in an objective portion on the basis of the digital data on the output voltage value information and the output current value information outputted from the A/D converter 27. When the control section 28 detects that the impedance value Z has exceeded a predetermined threshold value Zth, the control section 28 performs control for outputting high frequency power of a higher frequency on the waveform generation circuit 22 and the resonance circuit 23. Also, when the control section 28 detects that the impedance value Z is higher than predetermined threshold value Zth and the frequency of high frequency power outputted from the resonance circuit 23 is at the maximum, the control section 28 performs control for substantially stopping the output of high frequency power on the direct current power supply circuit 21, the waveform generation circuit 22 and the resonance circuit 23. The control section 28 in the present embodiment may also perform control for sounding a buzzer or the like (not shown) to inform an operator or the like that the output of high frequency power is substantially (or completely) stopped. A state where the output of high frequency power is substantially stopped means as a state where high frequency power having such a low level as not to cauterize a living tissue is being continuously outputted.

On the other hand, high frequency power (high frequency current and high frequency voltage) outputted from the high frequency power supply 2 is supplied to the treatment instrument 3 via the cable 31.

As shown in Fig. 1, the treatment instrument 3 is formed as a pair of forceps having two shanks connected so as to intersect with each other, and is configured to have the cable 31 extending from proximal end portions of the two shanks, the hand switch 32 electrically connected to the high frequency power supply 2 via a signal line, handles 33a and 33b respectively provided on the proximal end portions of the two shanks, and jaws 34a and 34b respectively provided on distal end portions of the two shanks. The treatment instrument 3 thus configured is capable of grasping a living tissue in an objective portion between the jaws 34a and 34b by operating the handles 33a and 33b so that the handles 33a and 33b are brought closer to each other.

High frequency power supplied to the treatment instrument 3 via the cable 31 is transmitted via conductors, not shown, respectively provided in the two shanks of the treatment instrument 3 in the lengthwise direction, and is thereafter outputted to the jaws 34a and 34b. When high frequency power is outputted to the jaws 34a and 34b in a state where a living tissue in an objective portion is grasped between the jaws 34a and 34b, the high frequency power is applied to the living tissue in the objective portion.

The operation of the electrosurgical apparatus 1 will now be described.

An operator brings the distal end portion of the treatment instrument 3 to a living tissue in an objective portion and holds the living tissue in the objective portion by grasping the living tissue between the jaws 34a and 34b by operating the handles 33a and 33b. In this state, the operator presses the hand switch 32 or the foot switch 4 to provide a command to output high frequency power from the high frequency power supply 2. With this operation, high frequency power is applied to the living tissue in the objective portion grasped between the jaws 34a and 34b.

As high frequency power is continuously applied to the living tissue in the objective portion, a phenomenon occurs in which, for example, as shown in Figs. 2 and 3, at a moment close to a point in time at which a vapor layer is formed in the living tissue, the impedance value Z increases with time t, while the value of power W applied to the living tissue decreases with time t.

On the other hand, the control section 28 determines whether or not the impedance value Z exceeds the predetermined threshold value Zth by monitoring at desired times digital data on output voltage value information and output current value information outputted from the A/D converter 27 (step S1 in Fig. 5). The control section 28 maintains the application of high frequency power to the living tissue in the objective portion as long as the impedance value Z does not exceed the predetermined threshold value Zth. When the control section 28 detects that the impedance value Z has exceeded the predetermined threshold value Zth, the control section 28 determines whether or not the frequency f of high frequency power presently outputted is the upper limit value fmax (step S2 in Fig. 5). It is assumed that in the present embodiment the predetermined threshold value Zth is set in advance as a value between 400 Ω and 1000 Ω, and that the predetermined threshold value Zth is higher than the impedance at which a change from constant-power control to constant-voltage control is made.

When the control section 28 detects that the frequency f of high frequency power is not the upper limit value fmax (step S2 in Fig. 5), the control section 28 performs control for increasing the frequency f one step on the waveform generation circuit 22 and the resonance circuit 23 (step S3 in Fig. 5). By this control, for example, as shown in Fig. 4, the frequency f of high frequency power is increased stepwise, as shown by fo → f₁, f₁ → f₂, and f₂ → f₃, at points in time (times t1, t2 and t3) at which the impedance value Z exceeds the predetermined threshold value Zth.

With consideration of an increase in electrical conductivity of the living tissue with the increase in frequency, for example, as shown in Figs. 2 and 3, the impedance value Z of the living tissue in the objective portion decreases at the points in time (times t1, t2 and t3) at which the frequency f of high frequency power is increased, and, simultaneously, the value of power W applied to the living tissue again is again increased to a value substantially equal to the power value at the initial stage (immediately after the start of application of high frequency power to the living tissue in the objective portion). The correlation between the frequency and the electric conductivity of the living tissue is, for example, as shown in the graph of Fig. 6.

The control section 28 repeats performing the above-described control for increasing stepwise the frequency f of high frequency power each time the impedance value Z exceeds the predetermined threshold value Zth. When the control section 28 detects (in step S2 in Fig. 5), at time t4, that the impedance value Z has finally exceeded the predetermined threshold value Zth (step S1 in Fig. 5) and the frequency f of high frequency power is the upper limit value fmax (e.g., f₃ shown in Fig. 4), the control section 28 performs control for substantially stopping the output of high frequency power on the direct current power supply circuit 21, the waveform generation circuit 22 and the resonance circuit 23. The control section 28 in the present embodiment may also perform control for sounding a buzzer or the like (not shown) to inform the operator or the like that the output of high frequency power is substantially stopped. The control section 28 in the present embodiment is not limited to the one that substantially stops the output of high frequency power at time t4. For example, the control section 28 may completely stop the output of high frequency power.

The control section 28 in the present embodiment is assumed to perform control for increasing the frequency f of high frequency power stepwise in the range of about 350 kHz to 5 MHz for example.

Also, a control section which performs control for increasing the frequency f of high frequency power from one step to another in a plurality of steps may suffice as the control section 28 in the present embodiment. The control section 28 is not limited to the one that increases the frequency f of high frequency power in the four steps corresponding to f₀, f₁, f₂ and f₃.

Further, the control section 28 in the present embodiment is not limited to the one that performs control for increasing the frequency f of high frequency power one step when detecting that the impedance value Z has exceeded the predetermined threshold value. For example, the control section 28 may perform control for increasing the frequency f of high frequency power one step when detecting that the value of power W applied to a living tissue is smaller than half the initial power value. Also, the control section 28 in the present embodiment may perform control for increasing the frequency f of high frequency power one step on the basis of either of the output voltage value information and the output current value information. Also, the control section 28 in the present embodiment may perform control for increasing the frequency f one step when detecting that the gradient of the impedance value Z with respect to passage of time has exceeded a predetermined threshold value.

On the other hand, the control section 28 in the present embodiment may perform control relating to high frequency induction heating for the purpose of obtaining an improved dehydration effect.

More specifically, the control section 28 in the present embodiment may further perform, in the sequential process shown in Fig. 5, control for outputting high frequency power having the frequency fmax for a predetermined time period after detecting that the impedance value Z has exceeded the predetermined threshold value Zth and the frequency f of high frequency power is the upper limit value fmax.

With consideration of the point that with the progress of dehydration a vapor layer is formed in a living tissue and the impedance of the living tissue increases, a situation can occur where the dehydration does not proceed further because the power applied to the living tissue decreases with the time.

Also, with consideration of the point that with the progress of dehydration a vapor layer is formed in a living tissue and the impedance of the living tissue increases, it is thought that the dehydrated state of the living tissue cannot be correctly detected by simply detecting the increase in impedance in the living tissue.

On the other hand, in the conventional art, the output power for example is only determined on the basis of the tissue impedance response in the very initial stage. Therefore, the conventional art has the problem that a living tissue in an objective portion is not sufficiently dehydrated and the joining strength in the objective portion is weak.

As described above, the electrosurgical apparatus 1 in the present embodiment is capable of continuously applying electric power of substantially the same value as that immediately after a start of application of high frequency power to a living tissue by increasing the frequency of the high frequency power stepwise with increase in impedance value of the living tissue. Consequently, the electrosurgical apparatus 1 in the present embodiment is capable of dehydrating a living tissue in an objective portion.

### (Second Embodiment)

Hereinafter, a high-frequency treatment apparatus and a high-frequency wave control method for use in a treatment for joining or coapting a living tissue such as digestive tract will be described by way of example.

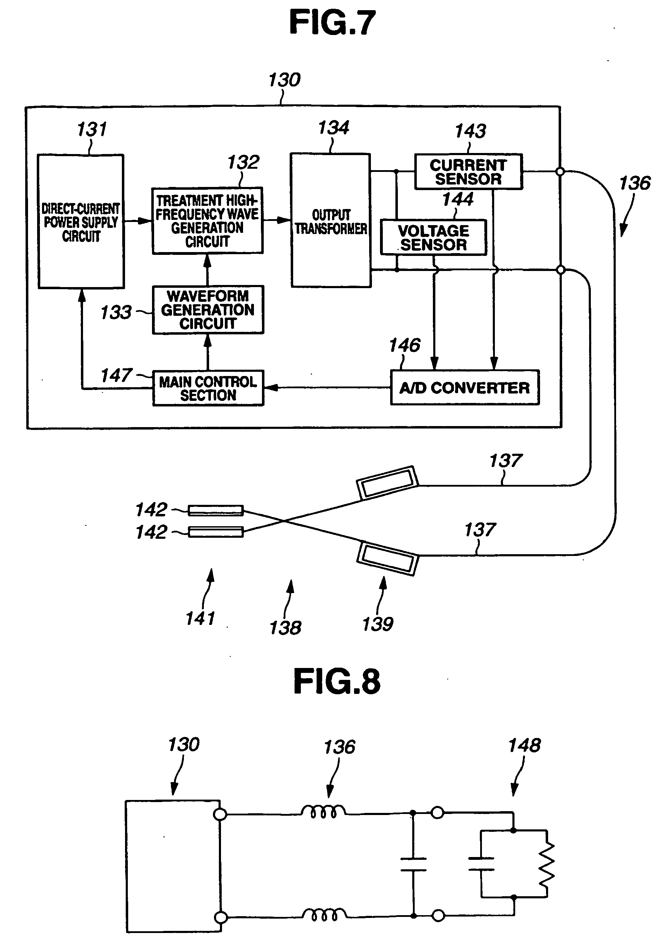

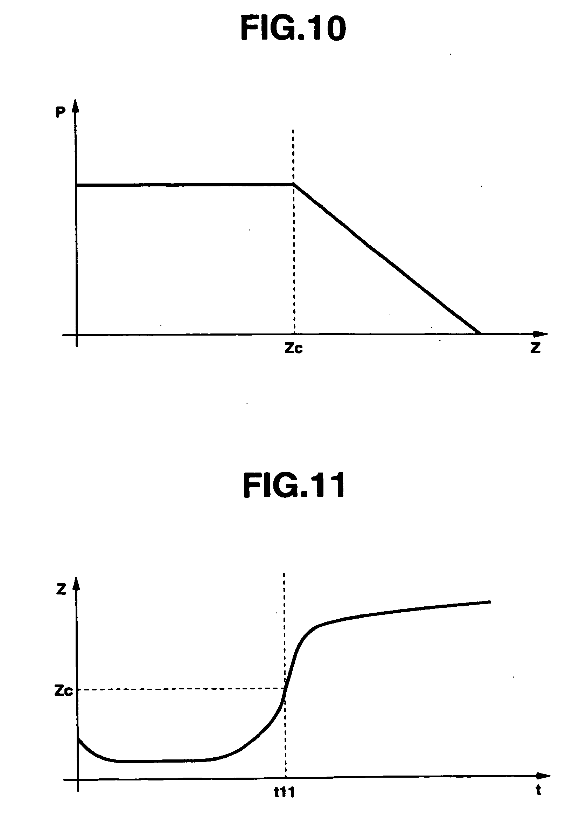

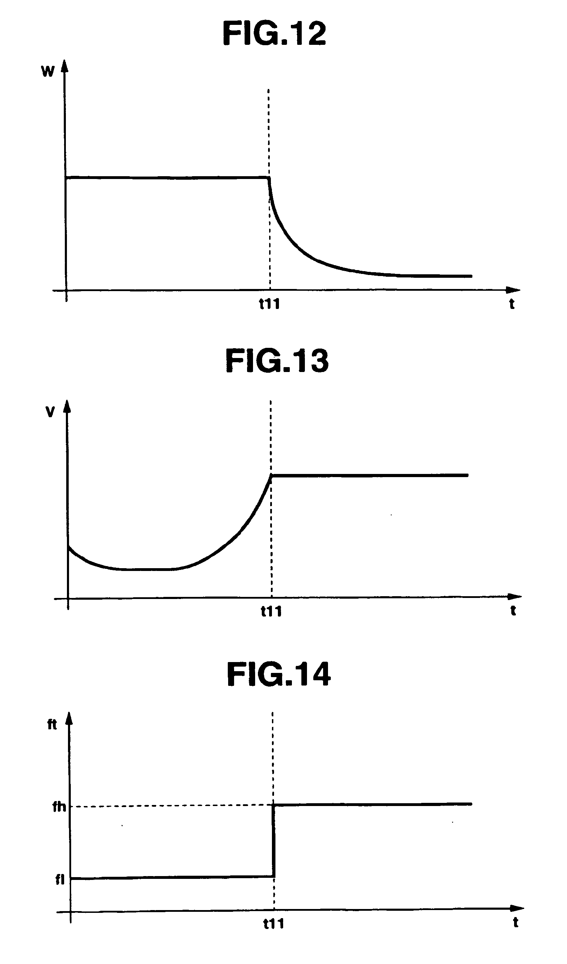

The second embodiment, however not forming part of the present invention, will be described referring to Figs. 7 to 15.

The high-frequency treatment apparatus will be described referring to Fig. 7.

A treatment high-frequency is used for treating a living tissue in a high-frequency treating system. That is, in a power supply 130, an electric power is supplied from a direct-current power supply circuit 131 to a treatment high-frequency wave generation circuit 132. The treatment high-frequency wave generation circuit 132 is controlled by a waveform generation circuit 133 to generate a treatment high-frequency wave and output the treatment high-frequency wave to an output transformer 134. The output transformer 134 is connected to a pair of output terminals. A pair of output terminals of the power supply 130 are connected respectively to a pair of signal lines 137 of a cable 136, and the pair of signal lines 137 of the cable 136 are respectively connected to a pair of electrodes 142 of a high-frequency treatment instrument 138. In this embodiment, the high-frequency treatment instrument 138 has the form of a forceps. Specifically, in the high-frequency treatment instrument 138, a par of jaws 141 on a distal end side of the instrument can be operated to be open and closed by operating a pair of handles 139 on a proximal end side of the instrument, and electrodes 142 are disposed on each closing side of the pair of jaws 141 to enable to hold a living tissue by the pair of electrodes 142. The living tissue can be treated by holding the living tissue by the pair of electrodes 142 and supplying the pair of electrodes 142 of the high-frequency treatment instrument 138 with the treatment high-frequency wave from the output transformer 134 of the power supply 130 through the pair of signal lines 137 of the cable 136 and applying the treatment high-frequency wave to the held living tissue from the pair of electrodes 142. Thus, a treatment high-frequency output unit is formed by the direct-current power supply circuit 131, the treatment high-frequency wave generation circuit 132 and the output transformer 134. A current sensor 143 and a voltage sensor 144 for respectively detecting a current value and a voltage value of the high-frequency wave outputted from the output transformer 134 are arranged between the output transformer 134 and the output terminals. The current value and the voltage value detected by the current sensor 143 and the voltage sensor 144 are A/D converted by an A/D converter 146 and outputted to a main control section 147. The main control section 147 computes an impedance of the living tissue from the inputted current and voltage values and controls the direct-current power supply circuit 131 and the waveform generation circuit 133 based on the computed impedance. In this manner, a detection unit is formed by the current sensor 143, the voltage sensor 144, the A/D converter 146 and the main control section 147, and a control unit is formed by the main control section 147 and the waveform generation circuit 133.

The high-frequency treatment method will be described referring to Fig.8 to Fig. 14.

Chemical properties of a coaptation treatment of a living tissue will be described.

It is considered that the coaptatin of the living tissue is established by hydrogen bond between polar groups of the tissue to be joined and has the same principle as adhesive. Specifically, protein of the living tissue is formed by peptide bond linkages of amino acids of a number of kinds and the peptide bond linkages respectively include polar groups such as NH, OH and a solid structure inherent to protein is formed by regular hydrogen bond of respective polar groups. Protein denaturing is caused by applying energy such as heat to the protein so that the hydrogen bond between the polar groups is dissociated. Then, the coaptation of the living tissue is performed by re-bonding the dissociated polar groups between the living tissues to be coapted. Here, water molecules tend to bond with the polar groups of the protein and in a case where moisture remains in the living tissue, the water molecules are bonded with the dissociated polar groups so that the hydrogen bond between the polar groups of the tissues to be coapted is inhibited. Thus, it is necessary to eliminate the water molecules from the living tissues by sufficiently dehydrating the living tissues in order to securely coapting the living tissues.

Referring to Fig. 8, electric properties of a living tissue 148 and the cable 136 will be described.

The living tissue 148 can be expressed as a parallel circuit of a resistance and a capacitor. With respect to cells of the living tissue 148, intracellular fluid and extracellular fluid have high conductance to constitute a resistance component of the living tissue 148. On the other hand, a cell membrane has insulation properties to constitute a capacitance component of the living tissue 148. An impedance based on the capacitance component is generally in inverse proportion to a frequency of a high-frequency wave. The impedance of the living tissue 148 is changed by applying energy to the living tissue 148. That is, the impedance is initially decreased by breakage of the cell membrane. Then, temperatures of the intracellular fluid and extracellular fluid are increased and motions of ions in the intracellular fluid and extracellular fluid are thus activated, so that the impedance is gently decreased, and subsequently the temperatures of the intracellular fluid and the extracellular fluid are further increased to commence dehydration of the intracellular fluid and the extracellular fluid, so that the impedance is gently increased. Thereafter, the intracellular fluid and the extracellular fluid are boiled and evaporated so that a vapor layer is generated. Since the vapor layer has insulation properties and constitutes a capacitance component, and of high impedance, the impedance of the living tissue is increased rapidly. A value of the impedance at which the generation of the vapor layer is considered to be started is determined in advance and the value is set to a switchover threshold value Zc. By further applying energy to the living tissue 148, the remaining moisture is successively boiled and evaporated so that the impedance gently increases to approach a fixed value. A value of the impedance at which the living tissue is considered to be completely dehydrated is determined in advance and the value is set to a final threshold value Ze.

The cable 136 can be expressed as combination of a coil in series with the living tissue 148 and a capacitance in parallel to the living tissue 148. Each of the signal lines 137 of the cable 136 has a predetermined inductance and constitutes an inductance component of the impedance of the cable 136. The impedance based on the inductance component is generally proportional to the frequency. Here, the cable 136 is relatively long, e.g. 3 meters or so in whole length and in a case where the frequency is specifically high, an influence of the inductance component increases. Also, a capacitor is formed by the both of signal lines 137 to constitute a capacitance component. The impedance on the basis of the capacitance component is generally in reverse proportion to the frequency.

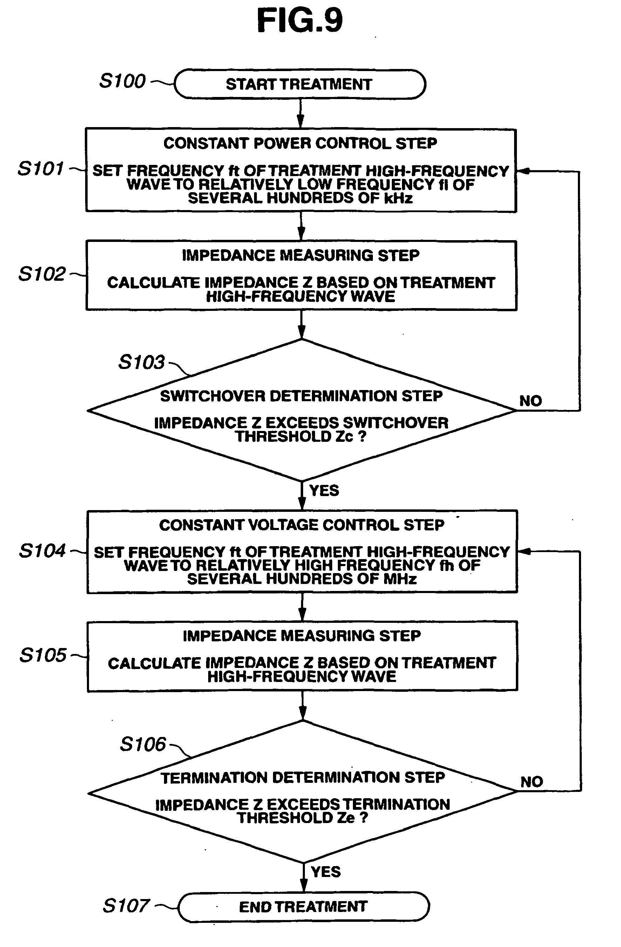

The high-frequency wave control method will be described referring to Fig.9 to Fig. 15.

### Treatment Start (S 100)

The output of treatment high-frequency wave is started.

At the start of the treatment, the resistance component is dominant in the living tissue and the living tissue is in a state where it can be regarded as a conductor, so that the impedance Z of the living tissue is relatively small.

### Constant Power Control Step (S101)

The current value and the voltage value of the treatment high-frequency wave outputted from the output transformer 134 are detected by the current sensor 143 and the voltage sensor 144 and the treatment high-frequency wave is controlled such that an electric power W of the treatment high-frequency wave outputted from the output transformer 134 is constant based on the detected current and voltage values. The frequency ft of the treatment high-frequency wave is set to a relatively low frequency fl, e.g. several hundreds of kHz. The high-frequency wave flows through the living tissue to generate Joule heat in the living tissue. Here, since the impedance Z of the living tissue is small, the high-frequency current is relatively large and since the calorific value Q by the Joule heat is proportional to the square of the current, relatively large energy is applied to the living tissue. By the application of the energy to the living tissue, after the breakage of cell membrane, the temperature rise of the intracellular fluid and the extracellular fluid, the intracellular fluid and the extracellular fluid are boiled and evaporated to generate the vapor layer, so that the impedance of the living tissue rapidly increases.

Besides, the reason why the frequency ft of the treatment high-frequency wave is not set to a relatively high frequency fh, e.g. several hundreds of MHz is as follows. Since the impedance of the cable 136 on the basis of the inductance component is generally proportional to the frequency, if the frequency ft of the treatment high-frequency wave is set to the relatively high frequency fh, the impedance of the cable 136 on the basis of the inductance component is made relatively high. In contrast thereto, the impedance on the basis of the resistance component of the living tissue is relatively small. Since the inductance component of the cable 136 and the resistance component of the living tissue, as such, are arranged in series, a power consumed by the cable 136 in the constant power W outputted from the power supply 130 is increased and a power consumed in the living tissue is decreased so that the Joule heat generated in the living tissue is decreased to lower the dehydration effect. On the other hand, the living tissue is in the state where it can be regarded as a conductor so that almost no heat is generated by the high-frequency induction heating and almost no dehydration effect by the high-frequency induction heating is obtained even if the frequency is set high.

### Impedance Measuring Step (S102)

The impedance Z of the living tissue to be treated is computed from the current value and the voltage value of the treatment high-frequency wave during the constant power control of the treatment high-frequency wave.

### Switchover Determination Step (S 103)

When the impedance Z computed based on the treatment high-frequency wave reaches the switchover threshold Zc, the control of the treatment high-frequency wave is switched from constant power control to the constant voltage control.

### Constant Voltage Control Step (S104)

The capacitance component is dominant in the living tissue where the vapor layer is generated and the living tissue is in a state where it is regarded as a dielectric material so that the impedance Z of the living tissue is relatively large. At the time when the vapor layer is generated in the living tissue and the impedance Z of the living tissue is rapidly increased, the intracellular fluid and the extracellular fluid still remain in the living tissue so that the dehydration of the living tissue is not complete. However, since the impedance Z of the living tissue is made large, the high-frequency current flowing through the living tissue is made small to lower the calorific value Q1 by the Joule heat. In particular, the impedance of the cable 136 on the basis of the capacitance component is generally in reverse proportion to the frequency of the high-frequency wave, the impedance of the cable 136 on the basis of the capacitance component is made relatively small. Since the capacitance component of the cable 136 and the capacitance component of the living tissue, as such, are arranged in parallel, the high-frequency current flowing through living tissue is further decreased so that the calorific value Q1 by the Joule heat is further decreased. Besides, it is necessary to make the voltage V of the treatment high-frequency wave not more than a fixed value in order to prevent generation of discharge at the electrodes 142, and there is a limitation on increase of the current flowing through the living tissue by increasing the voltage V. In view of the above, it is difficult to completely dehydrate the living tissue by the Joule heat.

In the constant voltage step, the treatment high-frequency wave outputted from the output transformer 134 is controlled so that the voltage V of the treatment high-frequency wave is constant. The frequency ft of the treatment high-frequency wave is set to a relatively high frequency fh, e.g. several MHz, which is higher than a frequency used in an ordinary high-frequency treatment apparatus. The living tissue is in a state where it can be regarded as a dielectric material and the living tissue is heated by the high-frequency induction heating. The calorific value Q2 by the high-frequency induction heating is proportional to a square of electric field intensity and the frequency and not influenced by a decrease of the current flowing through the living tissue, and is increased by the rise of the frequency. Further, since the impedance Z of the living tissue is made large, the influence of the inductance component of the cable 136 is relatively small, so that the voltage V applied to the living tissue is sufficiently secured and the electric field intensity is sufficiently secured. Therefore, relative large energy is applied to the living tissue and the intracellular fluid and the extracellular fluid remaining in the living tissue can be boiled and evaporated.

### Impedance Measurement Step (S105)

The impedance Z of the living tissue to be treated is computed from the current value and the voltage value of the treatment high-frequency wave during the constant voltage control of the treatment high-frequency wave.

### Termination Determination Step (S 106)

It is determined whether or not the impedance Z computed based on the treatment high-frequency wave reaches a termination threshold value Ze.

### Treatment Termination (S 107)

The output of the treatment high-frequency wave is stopped.

The high-frequency treatment apparatus and the high-frequency treatment method of the present embodiment have the following advantageous effects.

In the case where the impedance of the living tissue is smaller than the switchover threshold Zc, the living tissue is in a state where it is regarded as a conductor so that the current easily flows through the living tissue and therefore the calorific value Q1 by the Joule heat which is proportional to a square of the current is made relatively large. The influence of the inductance component of the cable 136 relative to the resistance component of the living tissue increases, so that a ratio of the power consumed by the cable 136 is increased and a ratio of the power consumed in the living tissue is decreased. In such case, the frequency ft of the treatment high-frequency wave is set to the relatively low frequency fl of several kHz to reduce the influence of the inductance component of the cable 136 to increase the ratio of the power consumed by the living tissue and the calorific value Q1 by the Joule heat and then the living tissue is treated mainly by the Joule heat. Thus, a sufficiently high dehydration effect is obtained. On the other hand, in a case where the impedance of the living tissue is larger than the switchover threshold Zc, the living tissue is in a state where it can be regarded as a dielectric material and the influence of the capacitance component of the cable 136 is made relatively large, so that the current is very hard to flow through the living tissue. Therefore, the calorific value Q1 by the Joule heat, which is proportional to a squire of the current, is made very small, whereas the calorific value Q2 by the high-frequency induction heating is made sufficiently large. In this case, the frequency ft of the treatment high-frequency wave is set to the relatively high frequency fh of several MHz to increase the calorific value Q2 by the high-frequency induction heating, which is proportional to the frequency, and then the living tissue is treated mainly by the high-frequency induction heating. Thus, a sufficiently high dehydration effect is obtained. Therefore, even in a case where the treatment high-frequency wave is transmitted by means of the cable 136, it is made possible to securely dehydrate the living tissue.

### (Third Embodiment)

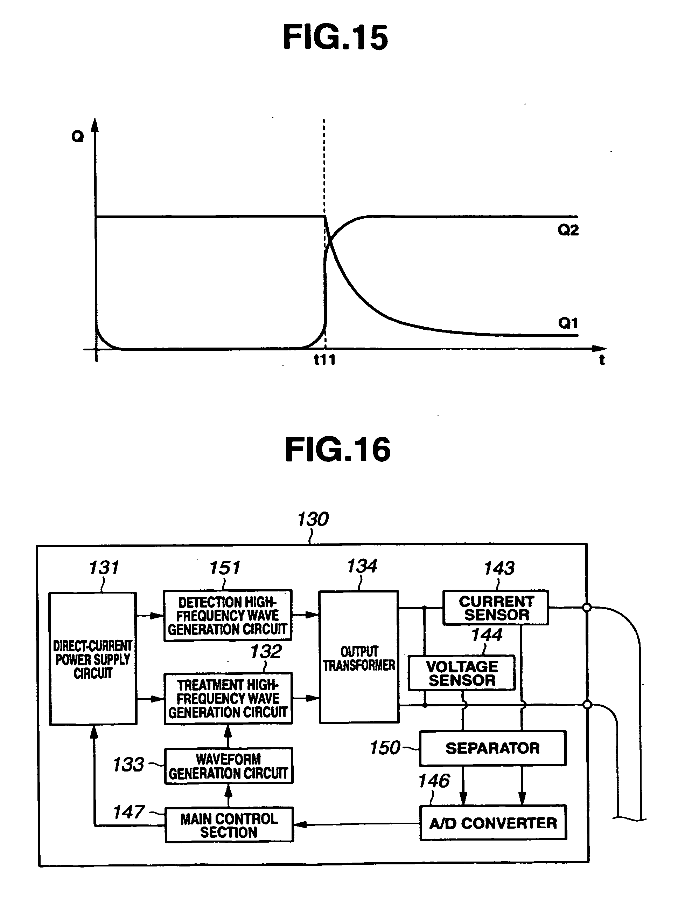

The third embodiment, however not forming part of the present invention, will be described referring to Figs. 16 to 19.

In this embodiment, a detection high-frequency wave of a relatively high frequency is used for measuring an impedance of the living tissue to be treated.

Referring to Fig. 16, the electric power is supplied from the direct-current power supply circuit 131 to a detection high-frequency wave generation circuit 151 in the power supply 130. The detection high-frequency wave generation circuit 151 generates a detection high-frequency wave and outputs the detection high-frequency wave to the output transformer 134. In the same manner as the treatment high-frequency wave, the detection high-frequency wave is supplied from the output transformer 134 to the pair of electrodes 142 through the pair of signal lines 137 of the cable 136, and applied to the living tissue held by the pair of electrodes 142. Thus, a detection high-frequency wave output unit is formed by the direct-current power supply circuit 131, the detection high-frequency wave generation circuit 151 and the output transformer 134. Values of the current and the voltage of the high-frequency wave outputted from the output transformer 134 are detected by the current sensor 143 and the voltage sensor 144 and separated by a separator 150 into a treatment high-frequency component and a detecting high-frequency component, and subjected to an A/D conversion by an A/D converter 146 and outputted to the main control section 147. The main control section 147 computes an impedance of the living tissue based on the current value and the voltage value of the detection high-frequency wave. As described, the detection unit of the present embodiment includes the separator 150. The main control section 147 controls the direct-current power supply circuit 131 and the waveform generation circuit 133 based on the computed impedance.

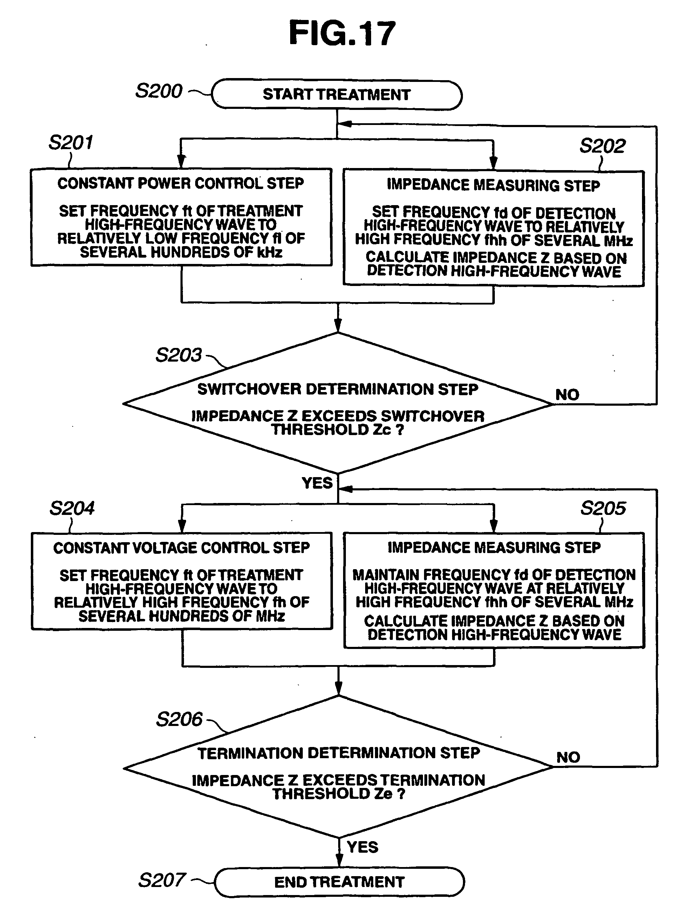

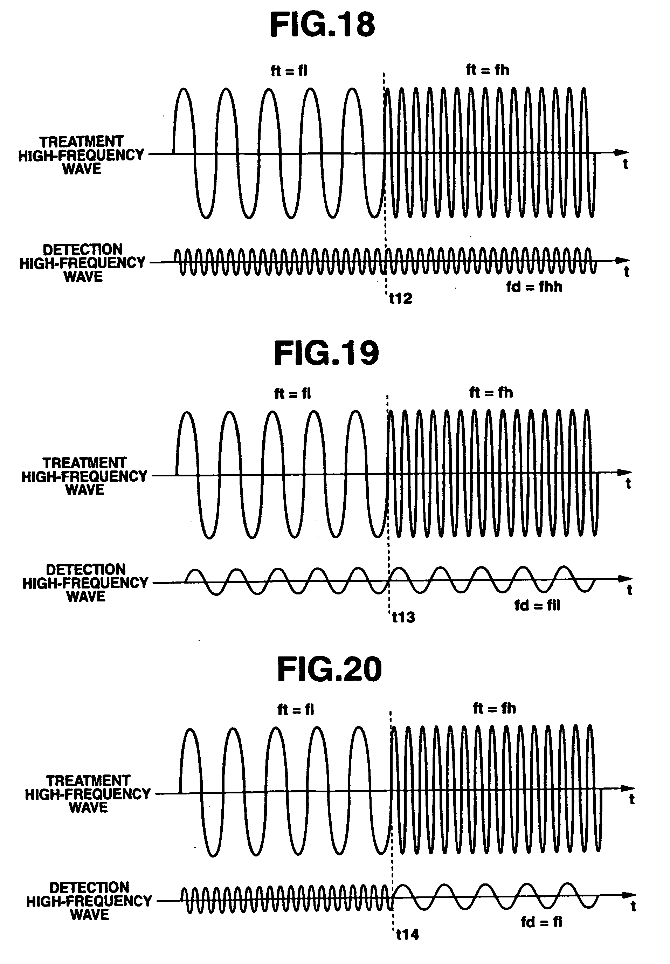

The high-frequency wave control method will be described referring to Fig. 17 and Fig. 18.

### Treatment Start (S200)

Outputs of the treatment high-frequency wave and the detection high-frequency wave are started.

### Constant Power Control Step (S201)

The constant power control is performed on the treatment high-frequency wave in the same manner as the second embodiment.

### Impedance Measurement Step (S202)

The impedance Z of the living tissue to be treated is computed from the current value and the voltage value of the detection high-frequency wave during the constant voltage control of the treatment high-frequency wave. As a voltage of the detection high-frequency wave, a weak voltage which is sufficiently smaller than the voltage of the treatment high-frequency wave is used. Besides, a frequency fd of the detection high-frequency wave is maintained to be constant and is set to a relatively high frequency fhh of several MHz which is equal to or higher than the relatively high frequency fh of several MHz of the treatment high-frequency wave. Besides, since the relatively high frequency fhh of several MHz is used as the detection high-frequency, the impedance on the basis of the inductance component of the cable 136 is made large, it is necessary to subtract the impedance on the basis of the inductance component of the cable 136 from the impedance directly computed from the current value and the voltage value of the detection high-frequency wave in order to compute the impedance Z of the living tissue. Besides, since the frequency fd of the detection high-frequency wave is maintained to be constant, the impedance on the basis of the inductance component of the cable 136 is made constant.

### Switchover Determination Step (S203)

When the impedance Z computed based on the detection high-frequency wave reaches the switchover threshold Zc, the control of the treatment high-frequency wave is switched from the constant power control to the constant voltage control.

### Constant Voltage Control Step (S204)

In the same manner as the second embodiment, the constant voltage control is performed on the treatment high-frequency wave.

### Impedance Measurement Step (S205)

The impedance Z of the living tissue to be treated is computed from the current value and the voltage value of the detection high-frequency wave during the constant voltage control of the treatment high-frequency wave. The frequency fd of the detection high-frequency wave is maintained as the relatively high frequency fhh of several MHz during the constant power control of the treatment high-frequency wave.

### Termination Determination Step (S206)

It is determined whether or not the impedance Z computed based on the treatment high-frequency wave reaches a termination threshold value Ze.

### Treatment Termination (S207)

The outputs of the treatment high-frequency wave and the detection high-frequency wave are stopped.

The high-frequency treatment apparatus and the high-frequency treatment method of the present embodiment have the following advantageous effects.

The impedance of the living tissue has frequency characteristics and the measured value of the impedance varies in dependence on the variation of the frequency of the high-frequency wave for detecting the impedance. In this embodiment, since the impedance is measured using the detection high-frequency wave of the constant frequency fhh over the whole treatment of the living tissue, it is made possible to measure the impedance while eliminating an influence of the variation of the measured value of the impedance caused by the variation of the frequency of the high-frequency wave for measuring the impedance.

In addition, as the frequency fd of the detection high-frequency wave, the relatively high frequency fhh of several MHz is used. In a case where the frequency fd of the detection high-frequency wave is high, the impedance on the basis of the capacitance component formed by the cell membrane and the vapor layer is measured small and the impedance measured is attributable to the impedance on the basis of the resistance component formed mainly by the intracellular fluid and the extracellular fluid. Therefore, even after the generation of the vapor layer is started, the impedance of the living tissue itself can be measured eliminating the influence of the vapor layer.

### (Fourth Embodiment)

The fourth embodiment, however not forming part of the present invention, will be described referring to Fig. 19.

In this embodiment, as the frequency fd of the detection high-frequency wave, a relatively low frequency fll which is equal to or lower than the relatively low frequency fl of several kHz of the treatment high-frequency wave is used in contrast to the third embodiment. In the case where the frequency fd of the detection high-frequency wave is low, the impedance on the basis of the capacitance component formed by the cell membrane and the vapor layer is measured large and the impedance measured is mainly attributable to the impedance on the basis of the conductance component. Therefore, it is made possible to detect the breakage of the cell membrane immediately after the application of the treatment high-frequency wave to the living tissue is started.

### (Fifth Embodiment)

The fifth embodiment, however not forming part of the present invention, will be described referring to Fig. 20.

In this embodiment, the main control section 147 computes an impedance of the living tissue based on the current value and the voltage value of the treatment high-frequency wave in the same manner as the second embodiment, and also computes an impedance of the living tissue based on the current value and the voltage value of the detection high-frequency wave. As the frequency fd of the detection high-frequency wave, a frequency different from the frequency ft of the treatment high-frequency wave is used. In this embodiment, in the case where the treatment high-frequency wave has the relatively low frequency fl of several kHz, the frequency fd of the detection high-frequency wave is set to a frequency equal to the relatively high frequency fh of several MHz of the treatment high-frequency wave, and in the case where the treatment high-frequency wave has the relatively high frequency fh of several MHz, the frequency fd of the detection high-frequency wave is set to a frequency equal to the relatively low frequency fl of several kHz of the treatment high-frequency wave. The main control section 147 determines whether or not the impedance of the living tissue reaches to the switchover threshold Zc based on the computed two impedances.

As described above, since the impedance of the living tissue which has the frequency characteristics is always measured based on the two high-frequency waves having frequencies different from each other, it is made possible to surely grasp the state of the living tissue. Particularly, in this embodiment, the relatively low frequency wave fl having the frequency of several kHz and the relatively high frequency wave fh having the frequency of several MHz are used, the detection of impedance of the living tissue itself and the detection of the breakage of the cell membrane are enabled.

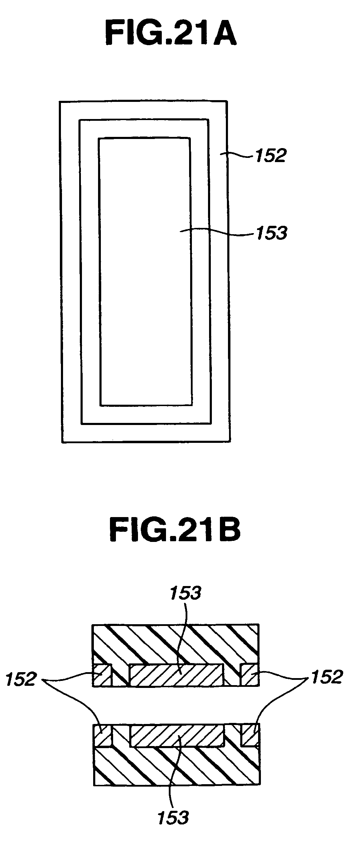

Hereinafter, reference embodiments, however not forming part of the present invention, will be described. A first reference embodiment will be described referring to Fig. 21A and Fig. 21B.

In this reference embodiment, edge electrodes 152 and central electrodes 153 are used as the electrodes 142 to be arranged at the jaws 141 of the high-frequency treatment instrument 138. As viewed in a contact direction with the living tissue, the edge electrode 152 is in a thin frame shape forming four sides of a rectangle, and the central electrode 153 is in a planner shape forming a rectangle and is arranged inside the edge electrode 152. The edge electrode 152 and the central electrode 153 are isolated with each other by an insulating member of a jaw body. When performing a coaptation treatment on the living tissue, the voltage is applied to a relatively narrow region between a pair of edge electrodes 152 so that the electric field intensity is made large in the region and the energy is applied to the region in a concentrated manner to improve the dehydration effect. Therefore, irrespective of thickness of the living tissue and the presence of the vapor layer, the living tissue is dehydrated evenly and sufficiently, so that even and sufficient coaptation strength is obtained. As a result, since the sufficient coaptation strength is obtained at the edge portions of the coaptation part, the coaptation part as a whole is hard to be cleaved.

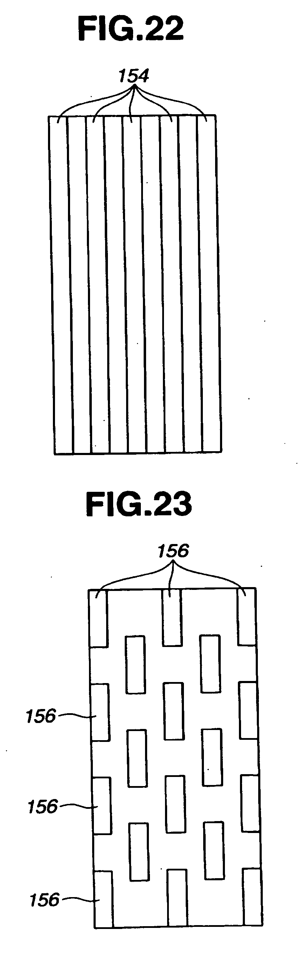

A second reference embodiment will be described referring to Fig. 22.

In this reference embodiment, as viewed in a contact direction with the living tissue, a plurality of bar electrodes 154 in the form of narrow sticks are arranged in parallel at constant intervals. In performing the coaptation treatment on the living tissue, the energy is applied to each narrow region between the pair of bar electrodes 154, which are facing each other, in a concentrated manner, so that even and sufficient coaptation strength in each region is obtained and the sufficient coaptation strength is obtained at the edge portions of the coaptation part. Further, even if the living tissue is cleaved at ends of the coaptation part on one side, the bar shaped coaptation part inside the cleaved coaptation part has even and sufficient coaptation strength and thus sufficient coaptation strength is obtained at new edge portions of the coaptation part, so that the coaptation part as a whole is hard to be cleaved.

A third reference embodiment will be described referring to Fig. 23.

In this reference embodiment, as viewed in a contact direction with the living tissue, a plurality of short bar electrodes 156 in the form of a narrow and short bar are aligned in the longitudinal direction thereof at constant intervals, lines of the plurality of short bar electrodes 156 are arranged in parallel with each other at constant intervals, and the short bar electrodes 156 in adjacent rows are alternately arranged to be shifted with respect to each other. In performing the coaptation treatment on the living tissue, the energy is applied to a narrow region between each pair of bar electrodes 154, which are facing each other, in a concentrated manner, so that even and sufficient coaptation strength in the region is obtained and the sufficient coaptation strength is obtained at the edge portions of the coaptation part. Further, since a broad non-dehydrated region which is not dehydrated remains at the coaptation part, regenerative ability of the living tissue is improved and exfoliation of the coaptation part is delayed and commence of the regeneration of the living tissue is facilitated.

The following is a description of a fourth reference embodiment.

In this reference embodiment, it is possible to supply the treatment high-frequency wave respectively and independently to the edge electrodes 152 and the central electrodes 153. With respect to the treatment high-frequency wave supplied to the edge electrodes 152, this wave is controlled in the same manner as the treatment high-frequency wave in the second embodiment. Specifically, until the impedance of the living tissue reaches the switchover threshold Zc, the treatment high-frequency is set to a relatively low frequency of several hundreds of kHz and after the impedance reaches the switchover threshold Zc, the treatment high-frequency is set to a relatively high frequency of several MHz. On the other hand, with respect to the treatment high-frequency wave supplied to the central electrodes 153, the frequency is set to a relatively low frequency of several hundreds of kHz over the whole coaptation treatment of the living tissue. In this reference embodiment, since the living tissue between the pair of edge electrodes 152 is dehydrated by the Joule heat and the high-frequency induction heating, the living tissue can be completely dehydrated and it is made possible to further enhance the coaptation strength.

## Claims

1. An electrosurgical apparatus (1) comprising:
a high frequency power generation section (2) configured to generate high frequency power to be applied to a living tissue;
a treatment instrument (3) with grasping members (34a, 34b) for grasping the living tissue therebetween;
the high frequency power generation section (2) comprising:
a voltage sensor (26) configured to detect a voltage of the high frequency power;
a current sensor (25) configured to detect a current of the high frequency power;
a control section (28) configured to compute the impedance of the living tissue between the grasping members (34a, 34b) on the basis of the voltage and the current, the control section (28) further configured to detect if a value of the
computed impedance exceeds a predetermined threshold,
**characterized in that**
the high frequency power generation section (2) further comprises a waveform generation circuit (22) configured to cause the high frequency power
generation section (2) to output a high frequency power having a first frequency or a high frequency power having a second frequency higher than the first frequency, and
the control section (28) is configured to switche an output from the waveform generation circuit (22) from the first frequency to the second frequency in response to detection of a value of the computed impedance exceeding the predetermined threshold.

2. The electrosurgical apparatus (1) according to claim 1, wherein in response to detection of a value of the computed impedance exceeding the predetermined threshold and the frequency being at a maximum, the control section (28) is configured to perform control for stopping or substantially stopping output of the high frequency power on the high frequency power generation section (2).

3. The electrosurgical apparatus (1) according to claim 1, wherein in response to detection of a value of the computed impedance exceeding the predetermined threshold and the frequency being at a maximum, the control section (28) is configured to perform control for outputting high frequency power having the maximum frequency for a predetermined time period on the high frequency power generation section (2).

4. The electrosurgical apparatus (1) according to claim 1, wherein in response to detection of a value of the computed impedance exceeding the predetermined threshold, the control section (28) is configured to perform control for increasing the frequency stepwise in the range from 350 kHz to 5 MHz on the high frequency power generation section (2).

## Patentansprüche

1. Elektrochirurgische Vorrichtung (1) aufweisend:
einen Abschnitt zum Erzeugen von Hochfrequenzenergie (2), der dazu eingerichtet ist, einem lebenden Gewebe zu beaufschlagende Hochfrequenzenergie zu erzeugen;
ein Behandlungsinstrument (3) mit Greifelementen (34a, 34b) zum Greifen des lebenden Gewebes zwischen diesen;
wobei der Abschnitt zum Erzeugen von Hochfrequenzenergie (2) aufweist:
einen Spannungssensor (26), der dazu eingerichtet ist, eine Spannung der Hochfrequenzenergie zu erfassen;
einen Stromsensor (25), der dazu eingerichtet ist, einen Strom der Hochfrequenzenergie zu erfassen;
einen Steuerungsabschnitt (28), der dazu eingerichtet ist, basierend auf der Spannung und dem Strom die Impedanz des lebenden Gewebes zwischen den Greifelementen (34a, 34b) zu berechnen, wobei der Steuerungsabschnitt (28) ferner dazu eingerichtet ist, zu erfassen, ob ein Wert der berechneten Impedanz einen vorbestimmten Schwellwert übersteigt,
**dadurch gekennzeichnet, dass**
der Abschnitt zum Erzeugen von Hochfrequenzenergie (2) ferner eine Wellenformerzeugungsschaltung (22) aufweist, die dazu eingerichtet ist, den Abschnitt zum Erzeugung von Hochfrequenzenergie (2) zu veranlassen, eine Hochfrequenzenergie mit einer ersten Frequenz oder eine Hochfrequenzenergie mit einer zweiten Frequenz, die größer als die erste Frequenz ist, auszugeben, und
der Steuerungsabschnitt (28) dazu eingerichtet ist, in Antwort auf die Erfassung eines Wertes der berechneten Impedanz, der den vorbestimmten Schwellwert überschreitet, eine Ausgabe der Wellenformerzeugungsschaltung (22) von der ersten Frequenz zu der zweiten Frequenz zu schalten.

2. Elektrochirurgische Vorrichtung (1) nach Anspruch 1, bei der der Steuerungsabschnitt (28) dazu eingerichtet ist, in Antwort auf die Erfassung eines Wertes der berechneten Impedanz, der den vorbestimmten Schwellwert überschreitet, und einem Maximum der Frequenz, eine Steuerung zum Beenden oder im Wesentlichen Beenden der Ausgabe der Hochfrequenzenergie an dem Abschnitt zum Erzeugen von Hochfrequenzenergie (2) auszuführen.

3. Elektrochirurgische Vorrichtung (1) nach Anspruch 1, bei der der Steuerungsabschnitt (28) dazu eingerichtet ist, in Antwort auf die Erfassung eines Wertes der berechneten Impedanz, der den vorbestimmten Schwellwert überschreitet, und einem Maximum der Frequenz, eine Steuerung zum Ausgeben der Hochfrequenzenergie mit der maximalen Frequenz für einen vorbestimmten Zeitabschnitt an dem Abschnitt zum Erzeugen von Hochfrequenzenergie (2) auszuführen.

4. Elektrochirurgische Vorrichtung (1) nach Anspruch 1, bei der der Steuerungsabschnitt (28) dazu eingerichtet ist, in Antwort auf die Erfassung eines Wertes der berechneten Impedanz, der den vorbestimmten Schwellwert überschreitet, eine Steuerung zum schrittweisen Erhöhen der Frequenz in dem Bereich von 350 kHz bis 5 MHz an dem Abschnitt zum Erzeugen von Hochfrequenzenergie (2) auszuführen.

## Revendications

1. Appareil électrochirurgical (1) comprenant :
une section (2) de génération d'énergie haute fréquence configurée pour générer une énergie haute fréquence destinée à être appliquée à un tissu vivant ;
un instrument (3) de traitement avec des éléments (34a, 34b) de préhension pour prendre le tissu vivant entre ceux-ci ;
la section (2) de génération d'énergie haute fréquence comprenant :
un détecteur (26) de tension configuré pour détecter une tension de l'énergie haute fréquence ;
un détecteur (25) de courant configuré pour détecter un courant de l'énergie haute fréquence ;
une section (28) de commande configurée pour calculer l'impédance du tissu vivant entre les éléments (34a, 34b) de préhension sur la base de la tension et du courant ;
la section (28) de commande configurée en outre pour détecter si une valeur de l'impédance calculée excède un seuil prédéterminé,
**caractérisé en ce que**
la section (2) de génération d'énergie haute fréquence comprend en outre un circuit (22) de génération de forme d'onde configuré pour faire en sorte que la section (2) de génération d'énergie haute fréquence délivre en sortie une énergie haute fréquence ayant une première fréquence ou une énergie haute fréquence ayant une deuxième fréquence supérieure à la première fréquence, et
la section (28) de commande est configurée pour commuter une sortie du circuit (22) de génération de forme d'onde de la première fréquence à la deuxième fréquence en réponse à une détection d'une valeur de l'impédance calculée excédant le seuil prédéterminé.

2. Appareil électrochirurgical (1) selon la revendication 1, dans lequel, en réponse à une détection d'une valeur de l'impédance calculée excédant le seuil prédéterminé et la fréquence étant à un maximum, la section (28) de commande est configurée pour exécuter une commande pour stopper ou sensiblement stopper la délivrance en sortie de l'énergie haute fréquence sur la section (2) de génération d'énergie haute fréquence.

3. Appareil électrochirurgical (1) selon la revendication 1, dans lequel, en réponse à une détection d'une valeur de l'impédance calculée excédant le seuil prédéterminé et la fréquence étant à un maximum, la section (28) de commande est configurée pour exécuter une commande pour délivrer en sortie une énergie haute fréquence ayant la fréquence maximum sur une période de temps prédéterminée sur la section (2) de génération d'énergie haute fréquence.

4. Appareil électrochirurgical (1) selon la revendication 1, dans lequel, en réponse à une détection d'une valeur de l'impédance calculée excédant le seuil prédéterminé, la section (28) de commande est configurée pour exécuter une commande pour augmenter la fréquence par pas dans la plage de 350 kHz à 5 MHz sur la section (2) de génération d'énergie haute fréquence.
